# FASCICULE DE BREVET EUROPEEN

(11) **EP 0 661 657 B1**
(45) Date de publication et mention de la délivrance du brevet: **19.09.2001**
(21) Numéro de dépôt: 94403044.4
(22) Date de dépôt: 28.12.1994
(51) Int. Cl.: G06F 19/00, H03K 19/0175

(54) **Procédé de configuration d'un dispositif implantable actif par ajustage de paramètres**
Verfahren zur Konfiguration einer implantierbaren aktiven Vorrichtung durch die Einstellung von Parametern
Configuration procedure of an implantable active device by adjusting parameters

(30) Priorité: 31.12.1993 FR 9315961
(43) Date de publication de la demande: 05.07.1995
(73) Titulaire: ELA MEDICAL, F-92541 Montrouge Cédex (FR)
(72) Inventeur: Dalmolin, Renzo, F-92320 Chatillon (FR); Pons, Pascal, F-38600 Fontaine (FR)
(74) Mandataire: Laget, Jean-Loup

(56) Documents cités:
- EP-A- 0 599 072
- US-A- 4 131 884
- US-A- 4 138 671
- US-A- 4 364 006
- US-A- 5 103 819

## Description

L'invention concerne un procédé de configuration d'un dispositif implantable actif par ajustage de paramètres.

Pour que leur fonctionnement soit satisfaisant, les dispositifs implantables actifs, tels que par exemple les stimulateurs cardiaques ou les défibrillateurs cardiaques, ont besoin de références électriques absolues précises, c'est-à-dire présentant par exemple des tensions de référence utilisables notamment pour la détection de l'activité cardiaque.

Par ailleurs, la miniaturisation des dispositifs implantables actifs impose le recours aux circuits intégrés. Les paramètres technologiques liés au processus de fabrication de ces circuits intégrés sont fluctuants d'un circuit à un autre ou d'un lot de production de circuits à un autre. Les fluctuations technologiques entraînent des fluctuations correspondantes de grandeurs électriques fournies par les circuits intégrés, telles que des fréquences d'horloge ou des tensions de référence, par exemple.

Afin de réduire les dispersions constatées, qui peuvent être importantes, il est nécessaire de procéder à un ajustage au cours du processus de fabrication du dispositif implantable actif.

Cet ajustage peut être externe, par action sur le circuit lui-même, ou interne, par action sur des éléments à mémoire.

L'ajustage externe peut consister en un ajustage dynamique de composants passifs extérieurs, puces résistives par exemple, qui sont ajustés au moyen d'un faisceau Laser ou d'une sableuse. Ce type d'ajustage présente plusieurs inconvénients. Tout d'abord, l'encombrement des appareils d'ajustage est excessif. Ensuite, il existe un risque d'interférences. Enfin, les types de composants ajustables se limitent essentiellement aux résistances.

L'ajustage externe peut aussi être réalisé par câblage, mais les inconvénients correspondants sont liés à l'encombrement et aux difficultés d'industrialisation.

L'ajustage interne peut être réalisé au moyen d'une mémoire vive de type RAM, qui présente l'inconvénient d'être sensible aux variations de tension d'alimentation et aux radiations.

Il peut aussi être réalisé au moyen d'une mémoire programmable, effaçable électriquement, de type EEPROM, dont l'inconvénient est la sensibilité aux radiations.

Une telle modification de la mémoire programmable de type EEPROM est décrite dans le document US 5.080.096. Une fois le stimulateur fabriqué, l'EEPROM est accessible par l'intermédiaire des bornes de sortie du stimulateur. L'EEPROM est programmée de l'extérieur pour ajuster le gain de la chaine de traitement d'un capteur d'activité. De la même façon le numéro de série peut être inscrit dans la mémoire de l'appareil.

US-A-4 138 671 décrit l'ajustage d'un convertisseur numérique - analogique intégré (DAC) au moyen de switches de type shorting zener diods. Après fabrication, le niveau de chaque circuit d'ajustage est sélectionné en fonction de la correction nécessaire pour l'étage du DAC correspondant, et la combinaison résultante de diodes zener est activée par claquage.

US-A-5 103 819 concerne un stimulateur cardiaque implantable. La partie amplification de détection, qui doit être protégée des chocs électriques inhérents à la défibrillation, comprend un amplificateur à contrôle automatique de gain (AGC), dans lequel une combinaison de switches est activée sélectivement par un microprocesseur pour contrôler le gain.

Un but de l'invention est de proposer un procédé de configuration par ajustage de paramètres électriques analogiques par des moyens de type numérique.

Un autre but de l'invention est de proposer un procédé de configuration d'un dispositif implantable actif par écriture et éventuellement codage de son numéro de série à des fins d'identification du dispositif.

La présente invention a pour objet un procédé de configuration d'un dispositif implantable actif par ajustage de paramètres au moyen d'un code, caractérisé en ce qu'il comporte : une étape de détermination du code de configuration, une étape d'écriture du code par claquage de diodes, et une étape de vérification de la validité du code.

Selon d'autres caractéristiques :
- l'étape d'écriture du code par claquage de diodes est réalisée par connexion à au moins une source de puissance extérieure au dispositif ;
- l'étape de détermination du code de configuration comprend la sélection du paramètre à ajuster, la lecture de sa valeur et la détermination, au moyen d'une table ou d'un logiciel, du code de configuration à écrire ;
- à chaque paramètre à ajuster est associé un mot binaire dont la longueur est fonction de la dispersion des valeurs lues du paramètre et de la précision souhaitée ;
- à chaque bit du mot binaire associé à un paramètre correspond une diode, dont l'état fonctionnel ou claqué correspond à un état, 0 ou 1, dudit bit ;
- l'étape d'écriture du code de configuration comprend le positionnement des adresses du bit à écrire, la connexion à au moins une source de puissance définissant une tension et une intensité de courant, l'écriture pendant une durée définie, et une vérification du claquage par lecture du bit de contrôle ;
- l'étape d'écriture comprend en outre, en cas de claquage non effectué, la réitération de l'opération d'écriture après augmentation de l'intensité de courant de source d'un incrément à chaque essai jusqu'au claquage effectif ;
- l'étape de vérification de la validité du code comprend la reconfiguration du dispositif implantable actif dans les conditions normales de fonctionnement et de contrôle de la validité de chacun des paramètres ;
- l'étape de détermination du code de configuration comprend en outre le forçage du code et la vérification de la bonne valeur du paramètre ;
- la tension de la source de puissance est de quelques V et l'intensité du courant de quelques mA ;
- la tension est de +9V par rapport à la source de puissance de polarité négative et l'intensité de 30mA ;
- l'incrément d'intensité du courant est de 10 mA ;
- l'augmentation de l'intensité du courant est limitée à une valeur prédéterminée ;
- la valeur prédéterminée de l'intensité du courant est 120 mA ;
- la diode est une diode Zener ;
- la diode est constituée par la jonction émetteur-base d'un transistor bipolaire dont la jonction collecteur-base est court-circuitée ;
- les étapes de détermination, d'écriture et de vérification du code de configuration, sont réalisables aussi bien en cours de fabrication qu'en fin de processus de fabrication, le boîtier du dispositif implantable actif étant ouvert, ou fermé, respectivement ;
- les étapes de détermination, d'écriture et de vérification du code de configuration, sont réalisables par télémétrie et par connexion à au moins une source de puissance extérieure au dispositif ;
- l'étape d'écriture du code met en oeuvre deux sources de puissance extérieures au dispositif ;
- l'une desdites sources est de tension négative et l'autre est de tension positive ;
- les sources de puissance extérieures sont reliées au connecteur du dispositif implantable actif destiné à recevoir les sondes.

L'invention a également pour objet l'application du procédé
- à la configuration fonctionnelle d'un stimulateur cardiaque ;
- à la configuration fonctionnelle d'un défibrillateur cardiaque ;
- à l'identification du dispositif implantable actif par codage de son numéro de série.

D'autres caractéristiques ressortent de la description qui suit, faite avec référence au dessin annexé sur lequel :

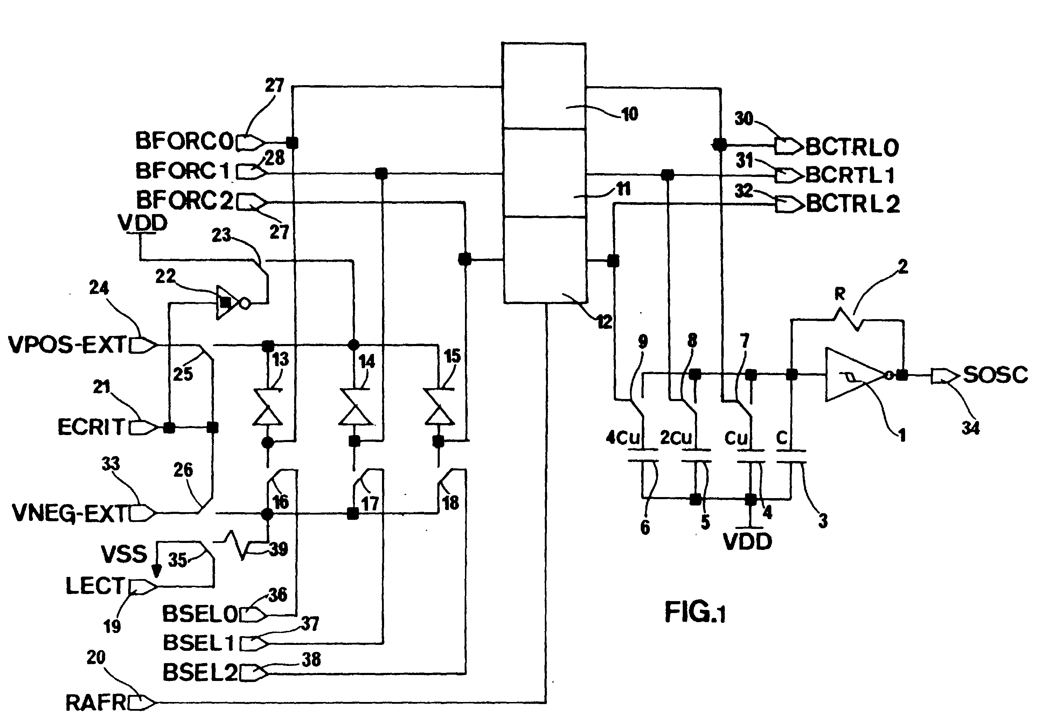

La figure 1 représente un schéma simplifié d'un exemple de réalisation de l'invention dans le cas d'un oscillateur dont la fréquence est ajustable par commutation de capacités.

Sur la figure 1, un oscillateur RC intégré est constitué d'un inverseur 1 avec hystérésis, d'une résistance en parallèle 2 de valeur R, et d'une capacité 3. En parallèle sur cette capacité 3 de valeur C, sont prévues trois capacités 4, 5, 6, de valeurs respectives Cu, 2Cu, 4Cu. Ces trois capacités 4-6 sont susceptibles d'être branchées en parallèle sur la capacité 3, ou mises hors circuit, au moyen de trois commutateurs 7, 8, 9 commandés chacun par un registre 10, 11, 12, respectivement.

Chacun de ces trois registres 10, 11, 12 est relié à une diode Zener, respectivement 13, 14, 15, qui se trouve en série avec un commutateur, respectivement 16, 17, 18.

Une borne commune 19 de lecture (LECT) permet d'assurer la lecture de l'état des diodes 13-15, et le test de vérification.

La borne 19 (LECT.), lorsqu'elle est activée, ferme un commutateur 35 qui, à travers une résistance 39, (par exemple de 90 KΩ afin de limiter le courant), relie à la tension négative commune VSS les commutateurs 16 à 18.

Une ligne 20 (RAFR) permet d'assurer le rafraîchissement cyclique des registres 10 à 12.

Les diodes 13 à 15 sont normalement reliées à la masse générale VDD par l'intermédiaire d'un commutateur 23. Le commutateur 23 est commandé par la ligne 21 (ECRIT) par l'intermédiaire d'un inverseur 22.

La ligne 21 (ECRIT) commande aussi, directement, deux commutateurs 25 et 26 reliant respectivement les diodes 13 à 15 à une source de puissance extérieure positive 24 (VPOS-EXT) et les commutateurs 16 à 18 à une source de puissance extérieure négative 33 (VNEG-EXT). Ainsi, lorsque le signal sur la ligne 21 (ECRIT) est bas (0) le commutateur 23 est fermé et les commutateurs 25 et 26 sont ouverts, et lorsque le signal est haut (1) le commutateur 23 est ouvert, et les commutateurs 25 et 26 sont fermés.

Les commutateurs 16 à 18 sont commandés par un bus de sélection, composé des lignes 36 (BSEL0), 37 (BSEL1) et 38 (BSEL2), afin d'activer la diode à claquer ou à vérifier.

Un bus de forçage, composé des lignes 27 (BFORC0), 28 (BFORC1) et 29 (BFORC2), permettent la simulation des codes définitifs dans les registres 10 à 12 et donc la vérification du code à appliquer.

Lorsque la ligne 21 (ECRIT.) applique un signal haut (1), le commutateur 23 s'ouvre, les commutateurs 25 et 26 se ferment, et les diodes 13 à 15 sont alimentées entre la source extérieure de puissance 24 (VPOS-EXT) et la source extérieure de puissance 33 (VNEG-EXT). Ces sources de puissance sont par exemple polarisées à +4V et -5V respectivement, de sorte que la diode 13 est soumise, lorsque la ligne 36 (BSEL0) est à un niveau haut 1, à une tension de 9V susceptible en principe d'assurer en quelques millisecondes le claquage de la diode 13.

Les registres 10 à 12 assurent la transmission des bits de forçage aux commutateurs 7 à 9 des capacités 4 à 6 de façon à assurer par exemple la mise en parallèle sur la capacité 3, de la capacité 4 correspondant au registre 10.

Les sorties des registres 10, 11, 12 sont reliées au bus de contrôle, composé respectivement des lignes 30 (BCTRL0), 31 (BCTRL1) et 32 (BCTRL2), permettant de procéder à l'étape de vérification de la validité du code de configuration.

Selon l'invention, à chaque paramètre électrique à ajuster, est associé un mot numérique de longueur prédéfinie, c'est-à-dire à N bits (3 bits dans l'exemple de la Figure 1). Ce nombre dépend de la dispersion du paramètre, spécifiée par le fondeur ou le concepteur, et de la précision souhaitée. A chaque bit du mot numérique est associée une diode Zener. Le code binaire associé à chaque diode est par convention : 0 pour une diode fonctionnelle, et 1 pour une diode claquée.

A l'état initial, avant écriture, toutes les diodes étant dans un état fonctionnel, les bits des codes binaires associés sont tous positionnés à la valeur 0 (arbitrairement).

L'étape d'écriture des codes d'ajustage, de programmation et d'identification se caractérise donc par le fait qu'il ne faut écrire que les bits de valeur 1.

Dans la pratique, une diode Zener est constituée par la jonction émetteur-base d'un transistor bipolaire dont la jonction collecteur-base est court-circuitée. Le claquage de la diode est effectué par élévation de la puissance, au moyen d'impulsions de courant d'intensité et de durée déterminées, par exemple quelques dizaines de mA, pendant quelques ms, sous quelques V. Dans le cas de la Figure 1, le claquage peut être effectué à 30 mA pendant 2 ms sous 9 V, pour chacune des diodes soumises au claquage. Lorsque le claquage est effectif, un court-circuit apparaît en raison de la diffusion de métallisation à travers la jonction. La diode est alors assimilable à une résistance de quelques KOhms.

Les codes d'ajustage sont donc obtenus lors de la polarisation des diodes. Cette polarisation est commutable au moyen de la ligne LECT afin de minimiser la consommation. Les codes sont mémorisés dans des registres, avec rafraîchissement cyclique par la ligne 20 (RAFR).

La procédure d'ajustage d'un paramètre électrique se compose de trois phases. Tout d'abord la détermination du code d'ajustage, dans une enceinte à régulation de température à 37°C, de préférence. Cette première phase comprend la sélection du paramètre analogique à contrôler ; la lecture de sa valeur ; la détermination, grâce à une table ou un logiciel, du code d'ajustage à écrire ; le forçage du code ; et la vérification de la valeur correcte du paramètre.

Ensuite, l'écriture du code par claquage de diodes. Dans cette deuxième phase, l'écriture du code se fait bit par bit, avec pour chaque paramètre à ajuster, une séquence des opérations comprenant : le forçage des alimentations internes de polarité négative à une tension négative externe (-5V, par exemple), le positionnement des adresses du bit à écrire, la connexion à la source de puissance de polarité positive (par exemple : V = + 4 V et I = 30mA), l'écriture pendant 2 ms, la déconnexion des sources de puissance, la vérification du claquage par lecture du bit de contrôle associé, et si le claquage n'est pas effectué, la réitération de l'opération d'écriture après augmentation du courant de source d'un incrément de 10 mA à chaque essai jusqu'au claquage effectif (avec toutefois une limite supérieure à l'intensité, de 120 mA par exemple).

Cette opération d'écriture du code par claquage de diode se fait pour chaque bit de valeur 1, par convention.

Enfin, la vérification de la validité du code après claquage. Cette troisième phase de la procédure d'ajustage comprend la reconfiguration du stimulateur dans les conditions normales de fonctionnement, c'est-à-dire avec alimentation de 2,8 V par exemple, et le contrôle de la validité de chacun des paramètres.

Dans l'exemple de réalisation de la Figure 1, une ou plusieurs des diodes 13 à 15 sont soumises au claquage. Les commutateurs correspondants 7 à 9 nécessaires à la commutation des capacités sont donc positionnés dans l'une des huit combinaisons possibles afin d'ajuster la fréquence de l'oscillateur.

Le claquage de diodes intégrées présente plusieurs avantages par rapport aux techniques antérieurement connues.

C'est un moyen d'ajustage interne, contrôlable de l'extérieur. Les diodes Zener sont intégrées dans la même puce que les autres fonctions analogiques ou mixtes du dispositif implantable actif, contribuant ainsi à un encombrement minimal. Ce principe d'ajustage n'entraîne pas d'accroissement sensible de la consommation. Son caractère irréversible assure une grande fiabilité dans le temps et une insensibilité aux perturbations extérieures comme les radiations ou les interférences électromagnétiques. L'ajustage peut prendre en compte les dispersions liées aussi bien aux circuits intégrés qu'aux composants discrets dans un environnement réaliste correspondant à la température du corps humain.

Le procédé d'ajustage de paramètres électriques selon l'invention est applicable dans un dispositif implantable actif tel qu'un stimulateur cardiaque ou un défibrillateur cardiaque pour assurer l'ajustage de fréquences d'horloge, de tensions et de courants de référence, ou de chaînes de traitement de signaux, par exemple pour l'ajustage de la sensibilité ou de la dynamique. Il est également applicable au tarage de chaînes de mesure, notamment de pile ou de sonde, à la programmation du modèle de stimulateur et à la sélection de ses modes de fonctionnement. Il est encore applicable au codage de l'identification du dispositif implantable actif, par exemple par son numéro de série. Cette dernière étape d'identification et de conformation du dispositif implantable actif peut intervenir en fin de processus de fabrication ou même plus tardivement, avant implantation.

Dans ce cas, toutes les opérations de détermination, d'écriture et de vérification des codes d'ajustage, de programmation et d'identification peuvent être réalisées par télémétrie, sans retrait de la pile. La connexion des sources extérieures s'effectue au moyen du connecteur de l'appareil destiné à recevoir les sondes.

## Revendications

1. Procédé de configuration d'un dispositif implantable actif par ajustage de paramètres au moyen d'un code, **caractérisé en ce qu'**il comporte, pour chaque paramètre à ajuster:
- une étape de détermination du code de configuration correspondant au dit paramètre,
- une étape d'écriture du code par claquage de diodes (13-15) intégrées dans le dispositif,
- et une étape de vérification de la validité du code.

2. Procédé selon la revendication 1, **caractérisé en ce que** l'étape d'écriture du code par claquage de diodes (13-15) est réalisée par connexion à au moins une source de puissance (24, 33) extérieure au dispositif.

3. Procédé selon la revendication 1, **caractérisé en ce que** l'étape de détermination du code de configuration comprend la sélection du paramètre à ajuster, la lecture de sa valeur et la détermination, au moyen d'une table ou d'un logiciel, du code de configuration à écrire.

4. Procédé selon la revendication 3, **caractérisé en ce que** à chaque paramètre à ajuster est associé un mot binaire dont la longueur est fonction de la dispersion des valeurs lues du paramètre et de la précision souhaitée.

5. Procédé selon la revendication 4, **caractérisé en ce que** : à chaque bit du mot binaire associé à un paramètre correspond une diode (13-15), dont l'état fonctionnel ou claqué correspond à un état, 0 ou 1, dudit bit.

6. Procédé selon la revendication 2, **caractérisé en ce que** l'étape d'écriture du code de configuration comprend le positionnement des adresses du bit à écrire, la connexion à au moins une source de puissance (24) définissant une tension et une intensité de courant, l'écriture pendant une durée définie, et une vérification du claquage par lecture du bit de contrôle.

7. Procédé selon la revendication 6, **caractérisé en ce que** l'étape d'écriture comprend en outre, en cas de claquage non effectué, la réitération de l'opération d'écriture après augmentation de l'intensité de courant de source d'un incrément à chaque essai jusqu'au claquage effectif.

8. Procédé selon la revendication 1, **caractérisé en ce que** l'étape de vérification de la validité du code comprend la reconfiguration du dispositif implantable actif dans les conditions normales de fonctionnement et de contrôle de la validité de chacun des paramètres.

9. Procédé selon la revendication 3, **caractérisé en ce que** l'étape de détermination du code de configuration comprend en outre le forçage du code et la vérification de la bonne valeur du paramètre.

10. Procédé selon la revendication 6, **caractérisé en ce que** la tension de la source de puissance (24, 33) est de quelques V et l'intensité du courant de quelques mA.

11. Procédé selon la revendication 10, **caractérisé en ce que** la tension est de +9V par rapport à la source de puissance (33) de polarité négative et l'intensité de 30mA.

12. Procédé selon la revendication 7, **caractérisé en ce que** l'incrément d'intensité du courant est de 10 mA.

13. Procédé selon la revendication 7, **caractérisé en ce que** l'augmentation de l'intensité du courant est limitée à une valeur prédéterminée.

14. Procédé selon la revendication 13, **caractérisé en ce que** la valeur prédéterminée de l'intensité du courant est 120 mA.

15. Procédé selon la revendication 5, **caractérisé en ce que** la diode (13-15) est une diode Zener.

16. Procédé selon la revendication 5, **caractérisé en ce que** la diode (13-15) est constituée par la jonction émetteur-base d'un transistor bipolaire dont la jonction collecteur-base est court-circuitée.

17. Procédé selon l'une des revendications 1 à 16, **caractérisé en ce que** les étapes de détermination, d'écriture et de vérification du code de configuration, sont réalisables aussi bien en cours de fabrication qu'en fin de processus de fabrication, le boîtier du dispositif implantable actif étant ouvert, ou fermé, respectivement.

18. Procédé selon l'une des revendications 1 à 6, **caractérisé en ce que** les étapes de détermination, d'écriture et de vérification du code de configuration, sont réalisables par télémétrie et par connexion à au moins une source de puissance extérieure au dispositif.

19. Procédé selon la revendication 2, **caractérisé en ce que** l'étape d'écriture du code met en oeuvre deux sources de puissance (24, 33) extérieures au dispositif.

20. Procédé selon la revendication 19, **caractérisé en ce que** l'une (33) desdites sources est de tension négative et l'autre (24) est de tension positive.

21. Procédé selon l'une des revendications 18 à 20, **caractérisé en ce que** les sources (24, 33) de puissance extérieures sont reliées au connecteur du dispositif implantable actif destiné à recevoir les sondes.

22. Application du procédé selon l'une des revendications 1 à 21 à la configuration fonctionnelle d'un stimulateur cardiaque.

23. Application du procédé selon l'une des revendications 1 à 21 à la configuration fonctionnelle d'un défibrillateur cardiaque.

24. Application du procédé selon l'une des revendications 1 à 21 à l'identification du dispositif implantable actif par codage de son numéro de série.

## Patentansprüche

1. Verfahren zur Konfiguration einer implantierbaren aktiven Vorrichtung durch die Einstellung von Parametern mittels eines Codes, **dadurch gekennzeichnet, dass** es für jeden einzustellenden Parameter umfasst:
einen Schritt zum Bestimmen des dem Parameter entsprechenden Konfigurationscodes,
einen Schritt zum Schreiben des Codes mit Hilfe des Durchbruchs von in der Vorrichtung integrierten Dioden (13-15)
und einen Schritt zum Prüfen der Gültigkeit des Codes.

2. Verfahren nach Anspruch 1, **dadurch gekennzeichnet, dass** der Schritt zum Schreiben des Codes mit Hilfe des Durchbruchs von Dioden (13-15) durch einen Anschluss an zumindest eine außerhalb der Vorrichtung liegende Energiequelle (24, 33) durchgeführt wird.

3. Verfahren nach Anspruch 1, **dadurch gekennzeichnet, dass** der Schritt zum Bestimmen des Konfigurationscodes die Auswahl des einzustellenden Parameters, das Lesen seines Wertes und die Bestimmung des zu schreibenden Konfigurationscodes mittels einer Tabelle oder eines Programms aufweist.

4. Verfahren nach Anspruch 3, **dadurch gekennzeichnet, dass** mit jedem einzustellenden Parameter ein Binärwort, dessen Länge von der Streuung der gelesenen Werte des Parameters und der gewünschten Genauigkeit abhängt, verbunden ist.

5. Verfahren nach Anspruch 4, **dadurch gekennzeichnet, dass** jedem Bit des mit einem Parameter verbundenen Binärwortes eine Diode (13-15) entspricht, deren funktioneller oder durchbrochener Zustand einem Zustand, 0 oder 1, des Bits entspricht.

6. Verfahren nach Anspruch 2, **dadurch gekennzeichnet, dass** der Schritt zum Schreiben des Konfigurationscodes eine Positionierung von Adressen des zu schreibenden Bits, den Anschluss an zumindest eine eine Spannung und eine Stromstärke definierende Energiequelle (24), das Schreiben während einer definierten Zeitdauer und eine Durchbruchsprüfung durch Lesen eines Kontrollbits umfasst.

7. Verfahren nach Anspruch 6, **dadurch gekennzeichnet, dass** der Schritt zum Schreiben außerdem, falls kein Durchbruch stattfindet, eine Wiederholung des Schreibvorganges nach einer Erhöhung der Stromstärke der Quelle um ein Inkrement bei jedem Versuch bis zu dem tatsächlichen Durchbruch aufweist.

8. Verfahren nach Anspruch 1, **dadurch gekennzeichnet, dass** der Schritt zum Prüfen der Gültigkeit des Codes eine Rekonfiguration der aktiven implantierbaren Vorrichtung bei normalen Betriebsbedingungen und eine Überprüfung der Gültigkeit jedes Parameters umfasst.

9. Verfahren nach Anspruch 3, **dadurch gekennzeichnet, dass** der Schritt zum Bestimmen des Konfigurationscodes außerdem ein Einprägen des Codes und die Prüfung des richtigen Wertes des Parameters umfasst.

10. Verfahren nach Anspruch 6, **dadurch gekennzeichnet, dass** die Spannung der Energiequelle (24, 33) einige V und die Stromstärke einige mA beträgt.

11. Verfahren nach Anspruch 10, **dadurch gekennzeichnet, dass** die Spannung +9V in Bezug auf die negative Polarität der Energiequelle (33) beträgt und die Stromstärke 30mA beträgt.

12. Verfahren nach Anspruch 7, **dadurch gekennzeichnet, dass** das Stromstärkeninkrement 10mA beträgt.

13. Verfahren nach Anspruch 7, **dadurch gekennzeichnet, dass** die Stromstärkenerhöhung auf einen vorbestimmten Wert begrenzt ist.

14. Verfahren nach Anspruch 13, **dadurch gekennzeichnet, dass** der vorbestimmte Stromstärkenwert 120 mA beträgt.

15. Verfahren nach Anspruch 5, **dadurch gekennzeichnet, dass** die Diode (13-15) eine Zenerdiode ist.

16. Verfahren nach Anspruch 5, **dadurch gekennzeichnet, dass** die Diode (13-15) einen Emitter-Basis-Übergang eines bipolaren Transistors umfasst, dessen Kollektor-Basis-Übergang kurzgeschlossen ist.

17. Verfahren nach einem der Ansprüche 1 bis 16, **dadurch gekennzeichnet, dass** die Schritte zum Bestimmen, zum Schreiben und zum Prüfen des Konfigurationscodes sowohl während der Herstellung als auch am Ende des Herstellungsprozesses durchgeführt werden können, wobei das Gehäuse der aktiven implantierbaren Vorrichtung jeweils offen bzw. geschlossen ist.

18. Verfahren nach einem der Ansprüche 1 bis 6, **dadurch gekennzeichnet, dass** die Schritte zum Bestimmen, zum Schreiben und zum Prüfen des Konfigurationscodes durch Telemetrie und durch Anschluss an zumindest eine außerhalb der Vorrichtung liegende Energiequelle durchgeführt werden können.

19. Verfahren nach Anspruch 2, **dadurch gekennzeichnet, dass** der Schritt zum Schreiben des Codes zwei außerhalb der Vorrichtung liegende Energiequellen (24, 33) verwendet.

20. Verfahren nach Anspruch 19, **dadurch gekennzeichnet, dass** eine (33) der Quellen eine negative Spannung aufweist und die andere (24) eine positive Spannung aufweist.

21. Verfahren nach einem der Ansprüche 18 bis 20, **dadurch gekennzeichnet, dass** die außen liegenden Energiequellen (24, 33) mit einem zum Empfangen von Sonden bestimmten Anschluss der aktiven implantierbaren Vorrichtung verbunden sind.

22. Anwendung des Verfahrens nach einem der Ansprüche 1 bis 21 auf die funktionelle Konfiguration eines Herzschrittmachers.

23. Anwendung des Verfahrens nach einem der Ansprüche 1 bis 21 auf die funktionelle Konfiguration eines Herzdefibrillators.

24. Anwendung des Verfahrens nach einem der Ansprüche 1 bis 21 auf die Identifizierung der aktiven implantierbaren Vorrichtung durch Kodierung ihrer Seriennummer.

## Claims

1. A process for configuring an active implantable device by adjusting parameters by means of a code, **characterised in that** it comprises, for each parameter to be adjusted:
- a stage of determining the configuration code corresponding to said parameter,
- a stage of writing the code by breakdown of diodes (13-15) incorporated in the device,
- and a stage of verifying the validity of the code.

2. A process according to claim 1, **characterised in that** the stage of the writing the code by breakdown of diodes (13-15) is performed by connection to at least one power source (24, 33) external to the device.

3. A process according to claim 1, **characterised in that** the stage of determining the configuration code comprises selection of the parameter to be adjusted, reading of the value thereof and determination, by means of a table or software, of the configuration code to be written.

4. A process according to claim 3, **characterised in that** each parameter to be adjusted is associated with a binary word, the length of which is a function of the dispersion of the read values of the parameter and of the desired precision.

5. A process according to claim 4, **characterised in that**: to each bit of the binary word associated with a parameter there corresponds a diode (13-15), the functional or broken-down state of which corresponds to one state, 0 or 1, of said bit.

6. A process according to claim 2, **characterised in that** the stage of writing the configuration code comprises positioning the addresses of the bit to be written, connection to at least one power source (24) defining a voltage and current intensity, writing for a defined period and verification of the breakdown by reading the check bit.

7. A process according to claim 6, **characterised in that** the writing stage further comprises, in the case of non-effected breakdown, reiteration of the writing operation after increasing the source current intensity by one increment per attempt until breakdown becomes effective.

8. A process according to claim 1, **characterised in that** the stage of verifying the validity of the code comprises reconfiguration of the active implantable device under normal operating conditions and normal conditions relating to checking of the validity of each of the parameters.

9. A process according to claim 3, **characterised in that** the stage of determining the configuration code further comprises setting of the code and verification of the correctness of the parameter.

10. A process according to claim 6, **characterised in that** the voltage of the power source (24, 33) is two or more V and the current intensity is two or more mA.

11. A process according to claim 10, **characterised in that** the voltage is +9V relative to the negative polarity power source (33) and the intensity is 30mA.

12. A process according to claim 7, **characterised in that** the current intensity increment is 10 mA.

13. A process according to claim 7, **characterised in that** the current intensity increase is limited to a preset value.

14. A process according to claim 13, **characterised in that** the preset value of the current intensity is 120 mA.

15. A process according to claim 5, **characterised in that** the diode (13-15) is a Zener diode.

16. A process according to claim 5, **characterised in that** the diode (13-15) consists of the emitter-base junction of a bipolar transistor, the collector-base junction of which is short-circuited.

17. A process according to one of claims 1 to 16, **characterised in that** the stages of determining, writing and verifying the configuration code may be performed either during manufacture or at the end of the manufacturing process, the case of the active implantable device being open or closed respectively.

18. A process according to one of claims 1 to 6, **characterised in that** the stages of determining, writing and verifying the configuration code may be performed by telemetry and by connection to at least one power source external to the device.

19. A process according to claim 2, **characterised in that** the stage of writing the code used two power sources (24, 33) external to the device.

20. A process according to claim 19, **characterised in that** one (33) of said sources is a negative voltage source and the other (24) is a positive voltage source.

21. A process according to one of claims 18 to 20, **characterised in that** the external power sources (24, 33) are connected to the connector of the active implantable device designed to receive probes.

22. Application of the process according to one of claims 1 to 21 to the functional configuration of a cardiac pacemaker.

23. Application of the process according to one of claims 1 to 21 to the functional configuration of a cardiac defibrillator.

24. Application of the process according to one of claims 1 to 21 to identification of the active implantable device by coding of its serial number.
